(19) Europäisches Patentamt
European Patent Office
Office européen des brevets

(11) EP 1 562 567 B1

(12) # EUROPÄISCHE PATENTSCHRIFT

(45) Veröffentlichungstag und Bekanntmachung des Hinweises auf die Patenterteilung:
**14.06.2017 Patentblatt 2017/24**

(21) Anmeldenummer: **03775368.8**

(22) Anmeldetag: **18.11.2003**

(51) Int Cl.:
***A61K 31/135*** (2006.01) ***A61K 31/415*** (2006.01)
***A61K 31/341*** (2006.01) ***A61P 29/00*** (2006.01)

(86) Internationale Anmeldenummer:
**PCT/EP2003/012882**

(87) Internationale Veröffentlichungsnummer:
**WO 2004/047823 (10.06.2004 Gazette 2004/24)**

(54) **KOMBINATION AUSGEWÄHLTER ANALGETIKA MIT COX II-INHIBITOREN**
COMBINATION OF SELECTED ANALGESICS AND COX-II INHIBITORS
ASSOCIATION D'ANALGESIQUES SELECTIONNES ET D'INHIBITEURS DE LA COX II

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **22.11.2002 DE 10254785**
**06.06.2003 DE 10326103**

(43) Veröffentlichungstag der Anmeldung:
**17.08.2005 Patentblatt 2005/33**

(60) Teilanmeldung:
**05027145.1 / 1 685 829**
**07014417.5 / 1 839 654**
**07023952.0 / 1 908 460**

(73) Patentinhaber: **Grünenthal GmbH**
**52078 Aachen (DE)**

(72) Erfinder:
- **SCHIENE, Klaus**
**41363 Jüchen (DE)**
- **HAASE, Günter**
**52159 Roetgen (DE)**
- **KÖGEL, Babette-Yvonne**
**52379 Langerwehe-Hamich (DE)**
- **FRIDERICHS, Elmar**
**52223 Stolberg (DE)**
- **JAHNEL, Ulrich**
**42855 Remscheid (DE)**

(56) Entgegenhaltungen:
**EP-A- 1 219 594** **WO-A-00/51685**
**WO-A-01/49654** **WO-A-02/28817**
**DE-A- 4 426 245** **DE-A- 19 525 137**
**US-A- 5 516 803**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft die Kombination aus Analgetika der Gruppe A mit COX II-Inhibitoren der Gruppe B, Arzneimittel, inclusive günstiger galenischer Formen, enthaltend diese Kombination und deren Verwendung insbesondere zur Behandlung von Schmerz.

Die Behandlung chronischer und nicht chronischer Schmerzzustände hat in der Medizin eine große Bedeutung. Zur Zeit besteht ein weltweiter Bedarf an zusätzlicher, nicht ausschließlich opioider, aber gut wirksamer Schmerztherapie. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für die Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Auch wenn die für die Schmerzbehandlung üblicherweise eingesetzten Analgetika - wie z.B. Opioiden, NA- und 5HT-Reuptake-Inhibitoren, NSAIDS wie auch COX-Inhibitoren - analgetisch wirksam sind, so treten doch - insbesondere bei den wirkstärkeren Opioiden - auch manchmal Nebenwirkungen auf.

Aufgabe der vorliegenden Erfindung war es daher, Stoffe oder Stoffkombinationen aufzufinden, die zur Behandlung von Schmerz geeignet sind und bei den wirksamen Dosen bevorzugt gleichzeitig geringere Nebenwirkungen zeigen als aus dem Stand der Technik bekannt, und bei den Kombinationen insbesondere einen synergistischen Effekt zur Behandlung der Schmerz zeigen.

Überraschenderweise wurde nun gefunden, daß eine Kombination aus ausgewählten Analgetika der Gruppe A mit COX II-Inhibitoren eine hervorragende analgetische Wirkung zeigen. Weiter erwiesen sich diese Kombinationen - deutlich über das Erwartete hinaus - bereits bei sehr geringen Dosen als so wirksam, daß die kombinierten Wirkstoffe niedrig dosiert eingesetzt werden konnten. Dadurch ist zu erwarten, daß sonst bei höheren notwendigen Dosierungen auftretende Nebenwirkungen deutlich zurückgehen werden.

Dementsprechend ist Erfindungsgegenstand eine Wirkstoffkombination aus wenigstens einer analgetischen **Verbindung A** und wenigstens einem **COX II-Inhibitor**, dadurch gekennzeichnet, dass der **COX II-Inhibitor** ausgewählt ist aus Celecoxib oder Rofecoxib, mit **Verbindung A** ausgewählt aus: (1 R,2R)-3-(3-Dimethylamino-1-ethyl-2-methyl-propyl)-phenol; Hydrochlorid und (1S,2S)-3-(3-Dimethylamino-1-ethyl-2-methyl-propyl)-phenol; Hydrochlorid,
in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

Der Geltungsbereich der Erfindung ist definiert durch die vorliegenden Ansprüche.

Die vorliegenden Beispiele, insofern sie sich auf andere als diese Verbindungen beziehen (d.h. Tests von Kombinationen mit den Verbindungen 1 und 4) sind Vergleichsbeispiele.

Offenbart wird auch eine Wirkstoffkombination aus wenigstens einer analgetischen **Verbindung A** und wenigstens einem **COX II-Inhibitor**, mit **Verbindung A** ausgewählt aus:

**Gruppe a)** enthaltend:

1-Phenyl-3-dimethylamino-propanverbindungen gemäß allgemeiner **Formel I**

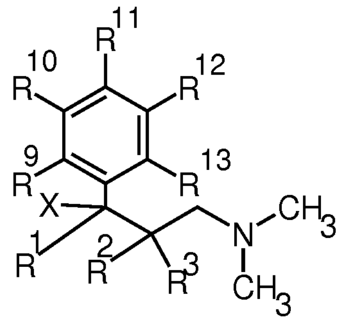

**I**

, worin

X ausgewählt ist aus OH, F, Cl, H oder $OC(O)R^7$ mit $R^7$ ausgewählt aus $C_{1-3}$-Alkyl, verzweigt oder unverzweigt, gesättigt oder ungesättigt, unsubstituiert oder ein- oder mehrfach substituiert,

$R^1$ ausgewählt ist aus $C_{1-4}$-Alkyl, verzweigt oder unverzweigt, gesättigt oder ungesättigt, unsubstituiert oder

ein- oder mehrfach substituiert,

$R^2$ und $R^3$ jeweils unabhängig voneinander ausgewählt sind aus H oder $C_{1-4}$-Alkyl, verzweigt oder unverzweigt, gesättigt oder ungesättigt, unsubstituiert oder ein- oder mehrfach substituiert,
oder
$R^2$ und $R^3$ zusammen einen gesättigten $C_{4-7}$-Cycloalkylrest bilden, unsubstituiert oder ein- oder mehrfach substituiert,

R9 bis $R^{13}$ jeweils unabhängig voneinander ausgewählt sind aus H, F, Cl, Br, I, $CH_2F$, $CHF_2$, $CF_3$, OH, SH, $OR^{14}$, $OCF_3$, $SR^{14}$, $NR^{17}R^{18}$, $SOCH_3$, $SOCF_3$; $SO_2CH_3$, $SO_2CF_3$, CN, $COOR^{14}$, $NO_2$, $CONR^{17}R^{18}$; $C_{1-6}$-Alkyl, verzweigt oder unverzweigt, gesättigt oder ungesättigt, unsubstituiert oder ein-oder mehrfach substituiert; Phenyl, unsubstituiert oder ein- oder mehrfach substituiert;

mit $R^{14}$ ausgewählt aus $C_{1-6}$-Alkyl; Pyridyl, Thienyl, Thiazolyl, Phenyl, Benzyl oder Phenethyl, jeweils unsubstituiert oder ein- oder mehrfach substituiert; $PO(O-C_{1-4}\text{-Alkyl})_2$, $CO(OC_{1-5}\text{-Alkyl})$, CONH-$C_6H_4$-($C_{1-3}$-Alkyl), CO($C_{1-5}$-Alkyl), CO-$CHR^{17}$-$NHR^{18}$, CO-$C_6H_4$-$R^{15}$, mit $R^{15}$ ortho-$OCOC_{1-3}$-Alkyl oder meta- oder para-$CH_2N(R^{16})_2$ mit $R^{16}$ $C_{1-4}$-Alkyl oder 4-Morpholino, wobei in den Resten $R^{14}$, $R^{15}$ und $R^{16}$ die Alkylgruppen verzweigt oder unverzweigt, gesättigt oder ungesättigt, unsubstituiert oder ein- oder mehrfach substituiert sein können;

mit $R^{17}$ und R18 jeweils unabhängig voneinander ausgewählt aus H; $C_{1-6}$-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,

oder

$R^9$ und $R^{10}$ oder $R^{10}$ und $R^{11}$ zusammen einen $OCH_2O$-, $OCH_2CH_2O$-, OCH=CH-, CH=CHO-, CH=C(CH3)O-, OC(CH3)=CH-, $(CH_2)_4$- oder OCH=CHO-Ring bilden, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;

in dargestellter Form oder in Form ihrer Säuren oder ihrer Basen oder in Form ihrer Salze, insbesondere der physiologisch verträglichen Salze, oder in Form ihrer Solvate, insbesondere der Hydrate;

**Gruppe b)** enthaltend:

substituierte 6-Dimethylaminomethyl-1-phenyl-cyclohexanverbindungen gemäß allgemeiner **Formel II**

**II**

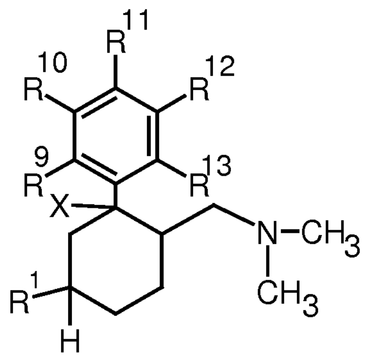

, worin

X ausgewählt ist aus OH, F, Cl, H oder $OC(O)R^7$ mit $R^7$ ausgewählt aus $C_{1-3}$-Alkyl, verzweigt oder unverzweigt, gesättigt oder ungesättigt, unsubstituiert oder ein- oder mehrfach substituiert,

$R^1$ ausgewählt ist aus $C_{1-4}$-Alkyl, Benzyl, $CF_3$, OH, $OCH_2$-$C_6H_5$, O-$C_{1-4}$-Alkyl, Cl oder F und
R9 bis $R^{13}$ jeweils unabhängig voneinander ausgewählt sind aus H, F, Cl, Br, I, $CH_2F$, $CHF_2$, $CF_3$, OH, SH, $OR^{14}$, $OCF_3$, $SR^{14}$, $NR^{17}R^{18}$, $SOCH_3$, $SOCF_3$; $SO_2CH_3$, $SO_2CF_3$, CN, $COOR^{14}$, $NO_2$, $CONR^{17}R^{18}$; $C_{1-6}$-Alkyl, verzweigt oder unverzweigt, gesättigt oder ungesättigt, unsubstituiert oder ein- oder mehrfach substituiert; Phenyl, unsubstituiert oder ein- oder mehrfach substituiert;

mit $R^{14}$ ausgewählt aus $C_{1-6}$-Alkyl; Pyridyl, Thienyl, Thiazolyl, Phenyl, Benzyl oder Phenethyl, jeweils unsubstituiert oder ein- oder mehrfach substituiert; $PO(O\text{-}C_{1-4}\text{-Alkyl})_2$, $CO(OC_{1-5}$-Alkyl), CONH-$C_6H_4$-($C_{1-3}$-Alkyl), CO($C_{1-5}$-Alkyl), CO-$CHR^{17}$-$NHR^{18}$, CO-$C_6H_4$-$R^{15}$, mit $R^{15}$ ortho-$OCOC_{1-3}$-Alkyl oder meta- oder para-$CH_2N(R^{16})_2$ mit $R^{16}$ $C_{1-4}$-Alkyl oder 4-Morpholino, wobei in den Resten $R^{14}$, $R^{15}$ und $R^{16}$ die Alkylgruppen verzweigt oder unverzweigt, gesättigt oder ungesättigt, unsubstituiert oder ein- oder mehrfach substituiert sein können;

mit $R^{17}$ und R18 jeweils unabhängig voneinander ausgewählt aus H; $C_{1-6}$-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,

oder
$R^9$ und R10 oder $R^{10}$ und $R^{11}$ zusammen einen $OCH_2O$-, $OCH_2CH_2O$-, OCH=CH-, CH=CHO-, CH=C(CH3)O-, OC(CH3)=CH-, $(CH_2)_4$- oder OCH=CHO-Ring bilden,

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze, oder in Form ihrer Solvate, insbesondere der Hydrate;;

**und/oder**

**Gruppe c)** enthaltend:

6-Dimethylaminomethyl-1-phenyl-cyclohexanverbindungen gemäß allgemeiner **Formel III**

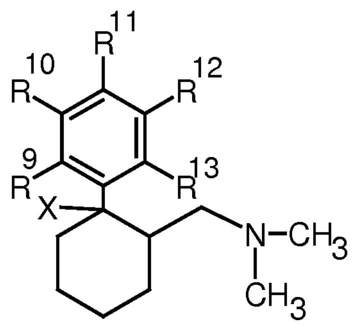

**III**

, worin

X ausgewählt ist aus OH, F, Cl, H oder $OC(O)R^7$ mit $R^7$ ausgewählt aus $C_{1-3}$-Alkyl, verzweigt oder unverzweigt, gesättigt oder ungesättigt, unsubstituiert oder ein- oder mehrfach substituiert, und

R9 bis $R^{13}$ jeweils unabhängig voneinander ausgewählt sind aus H, F, Cl, Br, I, $CH_2F$, $CHF_2$, $CF_3$, OH, SH, $OR^{14}$, $OCF_3$, $SR^{14}$, $NR^{17}R^{18}$, $SOCH_3$, $SOCF_3$; $SO_2CH_3$, $SO_2CF_3$, CN, $COOR^{14}$, $NO_2$, $CONR^{17}R^{18}$; $C_{1-6}$-Alkyl, verzweigt oder unverzweigt, gesättigt oder ungesättigt, unsubstituiert oder ein- oder mehrfach substituiert; Phenyl, unsubstituiert oder ein- oder mehrfach substituiert;

mit $R^{14}$ ausgewählt aus $C_{1-6}$-Alkyl; Pyridyl, Thienyl, Thiazolyl, Phenyl, Benzyl oder Phenethyl, jeweils unsubstituiert oder ein- oder mehrfach substituiert; $PO(O-C_{1-4}\text{-Alkyl})_2$, $CO(OC_{1-5}\text{-Alkyl})$, CONH-$C_6H_4$-($C_{1-3}$-Alkyl), CO($C_{1-5}$-Alkyl), CO-CHR$^{17}$-NHR$^{18}$, CO-$C_6H_4$-$R^{15}$, mit $R^{15}$ ortho-OCOC$_{1-3}$-Alkyl oder meta- oder para-$CH_2N(R^{16})_2$ mit $R^{16}$ $C_{1-4}$-Alkyl oder 4-Morpholino, wobei in den Resten $R^{14}$, $R^{15}$ und $R^{16}$ die Alkylgruppen verzweigt oder unverzweigt, gesättigt oder ungesättigt, unsubstituiert oder ein- oder mehrfach substituiert sein können;

mit $R^{17}$ und R18 jeweils unabhängig voneinander ausgewählt aus H; $C_{1-6}$-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,

oder

$R^9$ und $R^{10}$ oder $R^{10}$ und $R^{11}$ zusammen einen $OCH_2O$-, $OCH_2CH_2O$-, OCH=CH-, CH=CHO-, CH=C(CH3)O-, OC(CH3)=CH-, $(CH_2)_4$- oder OCH=CHO-Ring bilden,

mit der Maßgabe, daß, wenn $R^9$, $R^{11}$ und $R^{13}$ H entsprechen, und einer von $R^{10}$ oder $R^{12}$ H und der andere $OCH_3$ entspricht, X nicht OH sein darf,

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

Im Sinne der Offenbarung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht $C_{1-2}$-Alkyl für C1-oder C2-Alkyl, $C_{1-3}$-Alkyl für C1-, C2- oder C3-Alkyl, $C_{1-4}$-Alkyl für C1-, C2-, C3- oder C4-Alkyl, $C_{1-5}$-Alkyl für C1-, C2-, C3-, C4- oder C5-Alkyl, $C_{1-6}$-Alkyl für C1-, C2-, C3-, C4-, C5- oder C6-Alkyl, $C_{1-7}$-Alkyl für C1-, C2-, C3-, C4-, C5-, C6- oder C7-Alkyl, $C_{1-8}$-Alkyl für C1-, C2-, C3-, C4-, C5-, C6-, C7- oder C8-Alkyl, $C_{1-10}$-Alkyl für C1-, C2-, C3-, C4-, C5-, C6-, C7-, C8,- C9- oder C10-Alkyl und $C_{1-18}$-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht $C_{3-4}$-Cycloalkyl für C3- oder C4-Cycloalkyl, $C_{3-5}$-Cycloalkyl für C3-, C4-oder C5-Cycloalkyl, $C_{3-6}$-Cycloalkyl für C3-, C4-, C5- oder C6-Cycloalkyl, $C_{3-7}$-Cycloalkyl für C3-, C4-, C5-, C6- oder C7-Cycloalkyl, $C_{3-8}$-Cycloalkyl für C3-, C4-, C5-, C6-, C7- oder C8-Cycloalkyl, $C_{4-5}$-Cycloalkyl für C4- oder C5-Cycloalkyl, $C_{4-6}$-Cycloalkyl für C4-, C5- oder C6-Cycloalkyl, $C_{4-7}$-Cycloalkyl für C4-, C5-, C6- oder C7-Cycloalkyl, $C_{5-6}$-Cycloalkyl für C5- oder C6-Cycloalkyl und $C_{5-7}$-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, $CHF_2$, $CF_3$ oder $CH_2OH$ sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff substituiert im Sinne der Offenbarung die Substitution mindestens eines (gegebenenfalls auch mehrerer) Wasserstoffreste(s) durch F, Cl, Br, I, $NH_2$, SH, $OCH_3$, $SCH_3$, $N(CH_3)_2$, $NHCH_3$ oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von $CF_3$ oder an verschiedenen Stellen wie im Falle von -CH(OH)-CH=CH-$CHCl_2$. Besonders bevorzugte Substituenten sind hier F, Cl und OH. In Bezug auf Cycloalkyl kann der Wasserstoffrest auch durch $OC_{1-3}$-Alkyl oder $C_{1-3}$-Alkyl (jeweils ein- oder mehrfach substituiert oder unsubstituiert), insbesondere Methyl, Ethyl, n-Propyl, i-Propyl, $CF_3$, Methoxy oder Ethoxy, ersetzt sein.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ zu verstehen, unter $(CH_2)_{1-4}$ ist $-CH_2-$, $-CH_2-CH_2-$, $-CH_2-CH_2-CH_2-$ und $-CH_2-CH_2-CH_2-CH_2-$ zu verstehen, unter $(CH_2)_{4-5}$ ist $-CH_2-CH_2-CH_2-CH_2-$ und $-CH_2-CH_2-CH_2-CH_2-CH_2-$ zu verstehen, etc.

Unter einem Aryl-Rest werden Ringsysteme mit mindestens einem aromatischen Ring aber ohne Heteroatome in auch nur einem der Ringe verstanden. Beispiele sind Phenyl-, Naphthyl-, Fluoranthenyl-, Fluorenyl-, Tetralinyl- oder

Indanyl, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5]thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert die Substitution des Aryls oder Heteroaryls mit $R^{22}$, $OR^{22}$ einem Halogen, vorzugsweise F und/oder Cl, einem $CF_3$, einem CN, einem $NO_2$, einem $NR^{23}R^{24}$, einem $C_{1-6}$-Alkyl (gesättigt), einem $C_{1-6}$-Alkoxy, einem $C_{3-8}$-Cycloalkoxy, einem $C_{3-8}$-Cycloalkyl oder einem $C_{2-6}$-Alkylen.

Dabei steht der Rest $R^{22}$ für H, einen $C_{1-10}$-Alkyl-, vorzugsweise einen $C_{1-6}$-Alkyl-, einen Aryl- oder Heteroaryl- oder für einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen, die Reste $R^{23}$ und $R^{24}$, gleich oder verschieden, für H, einen $C_{1-10}$-Alkyl-, vorzugsweise einen $C_{1-6}$-Alkyl-, einen Aryl-, einen Heteroaryl- oder einen über $C_{1-3}$-Alkyl, gesättigt oder ungesättigt, oder eine $C_{1-3}$-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,

oder die Reste $R^{23}$ und $R^{24}$ bedeuten zusammen $CH_2CH_2OCH_2CH_2$, $CH_2CH_2NR^{25}CH_2CH_2$ oder $(CH_2)_{3-6}$, und der Rest $R^{25}$ für H, einen $C_{1-10}$-Alkyl-, vorzugsweise einen $C_{1-6}$-Alkyl-, einen Aryl-, oder Heteroaryl- Rest oder für einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydrol b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, $\alpha$-Liponsäure, Acetylglycin, Acetylsalicylsäure, Hippursäure und/oder Asparaginsäure. Besonders bevorzugt ist das Hydrochlorid-Salz und das Citrat.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1$\lambda^6$-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, $\alpha$-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit $NH_4^+$, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - verträglich ist. Besonders bevorzugt sind die Salze der Alkali- und Erdalkalimetalle aber auch $NH_4^+$, insbesondere aber (Mono-) oder (Di-) Natrium-,

(Mono-) oder (Di-) Kalium-, Magnesium- oder Calzium-Salze.

Verbindungen der **Gruppe a)** und deren Herstellung sind aus der DE 44 26 245 A1 bekannt. Verbindungen der **Gruppe b)** und **c)** und deren Herstellung sind aus der DE 195 25 137 A1 bekannt.

Unter COX II-Inhibitoren versteht man selektive Inhibitoren von COX II, der induzierbaren Isoform der Cyclooxygenase. Cyclooxygenase ist synonym für Prostaglandinendoperoxid-Synthase; ein Enzym, das die Funktion einer Dioxygenase u. einer Peroxidase in sich vereint u. - als Schlüsselenzym - die Umwandlung von Arachidonsäure in Prostaglandin H2 bzw. Peroxiden katalysiert. (s. Walter de Gruyter Pschyrembel 258. Auflage; Roche Lexikon Medizin, 4. Auflage). Weitere Informationen zu und insbesondere Auflistungen von COX II-Inhibitoren finden sich auf S. 13 bis 126 und insbesondere 21 bis 31 in "Analgesics, From Chemistry and Pharmacology to Clinical Application"; Editors Buschmann et al., 1. Aufl., Verlag Wiley-VCH, 2002. Der Inhalt dieses Buchabschnitts ist vollinhaltlich Teil der Beschreibung dieser Erfindung. Insbesondere versteht man unter COX II-Inhibitoren entsprechend beispielhaft Celecoxib, Rofecoxib, Etoricoxib, Valdecoxib, Parecoxib, Etodolac, Meloxicam oder Nimesulide. Besonders bedeutend ist das Celecoxib, das unter dem Namen LOSEC® bzw. Prilosec® oder CELEBREX® vertrieben wird, wie das Rofecoxib, das unter dem Namen VIOXX® vertrieben wird. Dabei wird unter selektiv entsprechend bevorzugt verstanden, daß die Verbindung eine stärkere Inhibition von COX II im Vergleich zu COX I zeigt und/oder insbesondere einen um den Faktor ≥ 5 kleineren $IC_{50}$ am COX II im Vergleich zu COX I zeigt.

Offenbart wird eine Kombination für die gilt, daß die **Verbindung A** in **Gruppe a**) ausgewählt ist aus Verbindungen gemäß **Formel I** für die gilt:

X ausgewählt ist aus OH, F, Cl, $OC(O)CH_3$ oder H, vorzugsweise OH, F, $OC(O)CH_3$ oder H,

**und/oder**

$R^1$ ausgewählt ist aus

$C_{1-4}$-Alkyl, gesättigt und unsubstituiert, verzweigt oder unverzweigt; vorzugsweise $CH_3$, $C_2H_5$, $C_4H_9$ oder t-Butyl, insbesondere $CH_3$ oder $C_2H_5$,

**und/oder**

$R^2$ und $R^3$ unabhängig voneinander ausgewählt sind aus

H, $C_{1-4}$-Alkyl, gesättigt und unsubstituiert, verzweigt oder unverzweigt; vorzugsweise H, $CH_3$, $C_2H_5$, i-Propyl oder t-Butyl, insbesondere H oder $CH_3$, vorzugsweise $R^3$ = H,

oder

$R^2$ und $R^3$ zusammen einen $C_{5-6}$-Cycloalkylrest bilden, gesättigt oder ungesättigt, unsubstituiert oder ein- oder mehrfach substituiert, vorzugsweise gesättigt und unsubstituiert, insbesondere Cyclohexyl.

**und/oder**

$R^9$ bis $R^{13}$, wobei 3 oder 4 der Reste $R^9$ bis $R^{13}$ H entsprechen müssen, unabhängig voneinander ausgewählt sind aus

H, Cl, F, OH, $CF_2H$, $CF_3$ oder $C_{1-4}$-Alkyl, gesättigt und unsubstituiert, verzweigt oder unverzweigt; $OR^{14}$ oder $SR^{14}$, mit $R^{14}$ ausgewählt aus $C_{1-3}$-Alkyl, gesättigt und unsubstituiert, verzweigt oder unverzweigt;

vorzugsweise H, Cl, F, OH, $CF_2H$, $CF_3$, $OCH_3$ oder $SCH_3$

oder $R^{12}$ und $R^{11}$ einen 3,4-OCH=CH-Ring bilden

insbesondere

wenn $R^9$, $R^{11}$ und $R^{13}$ H entsprechen, einer von $R^{10}$ oder $R^{12}$ auch H entspricht, während der andere ausgewählt ist aus:

Cl, F, OH, $CF_2H$, $CF_3$, $OR^{14}$ oder $SR^{14}$, vorzugsweise OH, $CF_2H$, $OCH_3$ oder $SCH_3$

oder,

wenn $R^9$ und $R^{13}$ H entsprechen und $R^{11}$ OH, $OCH_3$, Cl oder F, vorzugsweise Cl, entspricht, einer von $R^{10}$ oder $R^{12}$ auch H entspricht, während der andere OH, $OCH_3$, Cl oder F, vorzugsweise Cl, entspricht,

oder,

wenn $R^9$, $R^{10}$, $R^{12}$ und $R^{13}$ H entsprechen, $R^{11}$ ausgewählt ist aus $CF_3$, $CF_2H$, Cl oder F, vorzugsweise F,

oder,

wenn $R^{10}$, $R^{11}$ und $R^{12}$ H entsprechen, einer von $R^9$ oder $R^{13}$ auch H entspricht, während der andere ausgewählt ist aus OH, $OC_2H_5$ oder $OC_3H_7$.

Dabei gilt insbesondere für Verbindungen der **Gruppe a**), daß Verbindungen der **Formel I** mit $R^3$ = H in Form der Diastereomeren mit der relativen Konfiguration Ia

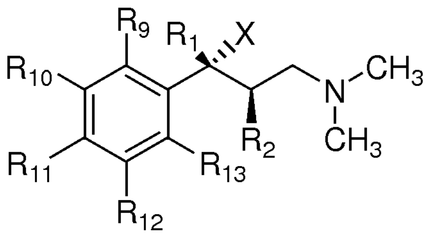

**Ia**

vorliegen, insbesondere in Mischungen mit höherem Anteil dieses Diastereomeren im Vergleich zum anderen Diastereomeren oder als reines Diastereomer verwendet werden.

Dabei gilt insbesondere für Verbindungen der **Gruppe a**), daß **Verbindung A** ausgewählt aus folgender Gruppe verwendet wird:

- (2RS,3RS)-1-Dimethylamino-3-(3-methoxy-phenyl)-2-methyl-pentan-3-ol,
- (+)-(2R,3R)-1 -Dimethylamino-3-(3-methoxy-phenyl)-2-methyl-pentan-3-ol,
- (2RS,3RS)-3-(3,4-Dichlorophenyl)-1-dimethylamino-2-methyl-pentan-3-ol,
- (2RS,3RS)-3-(3-Difluoromethyl-phenyl)-1-dimethylamino-2-methyl-pentan-3-ol,
- (2RS,3RS)-1-Dimethylamino-2-methyl-3-(3-methylsulfanylphenyl)-pentan-3-ol,
- (3RS)-1-Dimethylamino-3-(3-methoxy-phenyl)-4,4-dimethylpentan-3-ol,
- (2RS,3RS)-3-(3-Dimethylamino-1-ethyl-1-hydroxy-2-methylpropyl)-phenol,
- (1RS,2RS)-3-(3-Dimethylamino-1-hydroxy-1,2-dimethyl-propyl)-phenol,
- (+)-(1R,2R)-3-(3-Dimethylamino-1-hydroxy-1,2-dimethylpropyl)-phenol,
- (+)-(1R,2R)-3-(3-Dimethylamino-1 -hydroxy-1,2-dimethyl-propyl)-phenol,
- (-)-(1R,2R)-3-(3-Dimethylamino-1-ethyl-2-methyl-propyl)-phe-nol,
- (+)-(1 R,2R)-Essigsäure-3-dimethylamino-1-ethyl-1-(3-methoxy-phenyl)-2-methyl-propylester,
- (1RS)-1-(1-Dimethylaminomethyl-cyclohexyl)-1-(3-methoxyphenyl)-propan-1-ol,
- (2RS, 3RS)-3-(4-Chlorophenyl)-1-dimethylamino-2-methylpentan-3-ol,
- (+)-(2R,3R)-3-(3-Dimethylamino-1-ethyl-1-hydroxy-2-methylpropyl)-phenol,
- (2RS,3RS)-4-Dimethylamino-2-(3-methoxy-phenyl)-3-methyl-butan-2-ol und
- (+)-(2R,3R)-4-Dimethylamino-2-(3-methoxy-phenyl)-3-methyl-butan-2-ol,

vorzugsweise als Hydrochlorid.

Offenbart wird eine Kombination für die gilt, daß die **Verbindung A** in **Gruppe b**) ausgewählt ist aus Verbindungen gemäß **Formel II** für die gilt, daß:

X ausgewählt ist aus

OH, F, Cl, $OC(O)CH_3$ oder H, vorzugsweise OH, F oder H, insbesondere OH,

**und/oder**

$R^1$ ausgewählt ist aus

$C_{1-4}$-Alkyl, $CF_3$, OH, O-$C_{1-4}$-Alkyl, Cl oder F, vorzugsweise OH, $CF_3$ oder $CH_3$,

**und/oder**

$R^9$ bis $R^{13}$, wobei 3 oder 4 der Reste $R^9$ bis $R^{13}$ H entsprechen müssen, unabhängig voneinander ausgewählt sind aus

H, Cl, F, OH, $CF_2H$, $CF_3$ oder $C_{1-4}$-Alkyl, gesättigt und unsubstituiert, verzweigt oder unverzweigt; $OR^{14}$ oder $SR^{14}$, mit $R^{14}$ ausgewählt aus $C_{1-3}$-Alkyl, gesättigt und unsubstituiert, verzweigt oder unverzweigt;

vorzugsweise H, Cl, F, OH, $CF_2H$, $CF_3$, $OCH_3$ oder $SCH_3$

oder $R^{12}$ und $R^{11}$ einen 3,4-OCH=CH-Ring bilden,

insbesondere

wenn $R^9$, $R^{11}$ und $R^{13}$ H entsprechen, einer von $R^{10}$ oder $R^{12}$ auch H entspricht, während der andere ausgewählt ist aus:

Cl, F, OH, $CF_2H$, $CF_3$, $OR^{14}$ oder $SR^{14}$, vorzugsweise OH, $CF_2H$, $OR^{14}$ oder $SCH_3$, insbesondere OH oder $OC_{1-3}$-Alkyl, vorzugsweise OH oder $OCH_3$,

oder,

wenn $R^9$ und $R^{13}$ H entsprechen und $R^{11}$ OH, $OCH_3$, Cl oder F, vorzugsweise Cl, entspricht, einer von $R^{10}$ oder $R^{12}$ auch H entspricht, während der andere OH, $OCH_3$, Cl oder F, vorzugsweise Cl, entspricht,

oder,

wenn $R^9$, $R^{10}$, $R^{12}$ und $R^{13}$ H entsprechen, $R^{11}$ ausgewählt ist aus $CF_3$, $CF_2H$, Cl oder F, vorzugsweise F,

oder,

wenn $R^{10}$ , $R^{11}$ und $R^{12}$ H entsprechen, einer von $R^9$ oder $R^{13}$ auch H entspricht, während der andere ausgewählt ist aus OH, $OC_2H_5$ oder $OC_3H_7$,

ganz insbesondere bevorzugt,

wenn $R^9$, $R^{11}$ und $R^{13}$ H entsprechen, einer von $R^{10}$ oder $R^{12}$ auch H entspricht, während der andere ausgewählt ist aus:

Cl, F, OH, SH, $CF_2H$, $CF_3$, $OR^{14}$ oder $SR^{14}$, vorzugsweise OH oder $OR^{14}$, insbesondere OH oder $OC_{1-3}$-Alkyl, vorzugsweise OH oder $OCH_3$.

Dabei gilt insbesondere für Verbindungen der **Gruppe b)**, daß Verbindungen der **Formel II** in Form der Diastereomeren mit der relativen Konfiguration IIa

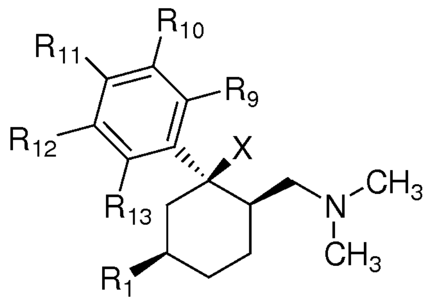

**IIa**

vorliegen, insbesondere in Mischungen mit höherem Anteil dieses Diastereomeren im Vergleich zum anderen Diastereomeren oder als reines Diastereomer verwendet werden.

Dabei gilt insbesondere für Verbindungen der **Gruppe b**), daß **Verbindung A** ausgewählt aus folgender Gruppe verwendet wird:

- (1 RS,3RS,6RS)-6-Dimethylaminomethyl-1-(3-methoxyphenyl)-cyclohexan-1,3-diol,
- (+)-(1 R,3R,6R)-6-Dimethylaminomethyl-1-(3-methoxyphenyl)-cyclohexan-1,3-diol,
- (1 RS,3RS,6RS)-6-Dimethylaminomethyl-1-(3-hydroxyphenyl)-cyclohexan-1,3-diol,
- (1 RS,3SR,6RS)-6-Dimethylaminomethyl-1-(3-methoxyphenyl)-cyclohexan-1,3-diol,
- (+)-(1 R,2R,5S)-3-(2-Dimethylaminomethyl-1-hydroxy-5-methyl-cyclohexyl)-phenol oder
- (1 RS,2RS,5RS)-3-(2-Dimethylaminomethyl-1-hydroxy-5-tri-fluoromethyl-cyclohexyl)-phenol,

vorzugsweise als Hydrochlorid.

Offenbart wird eine Kombination für die gilt, daß die **Verbindung A** in **Gruppe c**) ausgewählt ist aus Verbindungen gemäß **Formel III** für die gilt, daß:

X ausgewählt ist aus

OH, F, Cl, $OC(O)CH_3$ oder H, vorzugsweise OH, F oder H, insbesondere F oder H.

**und/oder**

$R^9$ bis $R^{13}$, wobei 3 oder 4 der Reste $R^9$ bis $R^{13}$ H entsprechen müssen, unabhängig voneinander ausgewählt sind aus

H, Cl, F, OH, $CF_2H$, $CF_3$ oder $C_{1-4}$-Alkyl, gesättigt und unsubstituiert, verzweigt oder unverzweigt; $OR^{14}$ oder $SR^{14}$, mit $R^{14}$ ausgewählt aus $C_{1-3}$-Alkyl, gesättigt und unsubstituiert, verzweigt oder unverzweigt;

vorzugsweise H, Cl, F, OH, $CF_2H$, $CF_3$, $OCH_3$ oder $SCH_3$

oder $R^{12}$ und $R^{11}$ einen 3,4-OCH=CH-Ring bilden

insbesondere dadurch gekennzeichnet, daß,

wenn $R^9$, $R^{11}$ und $R^{13}$ H entsprechen, einer von $R^{10}$ oder $R^{12}$ auch H entspricht, während der andere ausgewählt ist aus:

Cl, F, OH, $CF_2H$, $CF_3$, $OR^{14}$ oder $SR^{14}$, vorzugsweise OH, $CF_2H$, $OR^{14}$ oder $SCH_3$, insbesondere OH oder $OC_{1-3}$-Alkyl, vorzugsweise OH oder $OCH_3$,

oder,

wenn $R^9$ und $R^{13}$ H entsprechen und $R^{11}$ OH, $OCH_3$, Cl oder F, vorzugsweise Cl, entspricht, einer von $R^{10}$ oder $R^{12}$ auch H entspricht, während der andere OH, $OCH_3$, Cl oder F, vorzugsweise Cl, entspricht,

oder,

wenn $R^9$, $R^{10}$, $R^{12}$ und $R^{13}$ H entsprechen, $R^{11}$ ausgewählt ist aus $CF_3$, $CF_2H$, Cl oder F, vorzugsweise F,

oder,

wenn $R^{10}$, $R^{11}$ und $R^{12}$ H entsprechen, einer von $R^9$ oder $R^{13}$ auch H entspricht, während der andere ausgewählt ist aus OH, $OC_2H_5$ oder $OC_3H_7$,

ganz insbesondere bevorzugt,

wenn $R^9$, $R^{11}$ und $R^{13}$ H entsprechen, einer von $R^{10}$ oder $R^{12}$ auch H entspricht, während der andere ausgewählt ist aus:

Cl, F, OH, SH, $CF_2H$, $CF_3$, $OR^{14}$ oder $SR^{14}$, vorzugsweise OH oder $OR^{14}$, insbesondere OH oder $OC_{1-3}$-Alkyl, vorzugsweise OH oder $OCH_3$.

Dabei gilt insbesondere für Verbindungen der **Gruppe c**), daß Verbindungen der **Formel III** in Form ihrer Diastereomeren mit der relativen Konfiguration IIIa

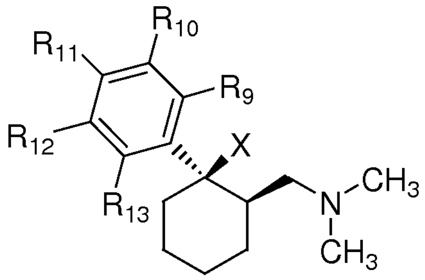

**IIIa**

vorliegen, insbesondere in Mischungen mit höherem Anteil dieses Diastereomeren im Vergleich zum anderen Diastereomeren oder als reines Diastereomer verwendet werden.

Dabei gilt insbesondere für Verbindungen der **Gruppe c),** daß **Verbindung A** ausgewählt aus folgender Gruppe verwendet wird:

- (+)-(1 R,2R)-3-(2-Dimethylaminomethyl-1-fluoro-cyclohexyl)-phenol,
- (+)-(1 S,2S)-3-(2-Dimethylaminomethyl-cyclohexyl)-phenol oder
- (-)-(1R,2R)-3-(2-Dimethylaminomethyl-cyclohexyl)-phenol,

vorzugsweise als Hydrochlorid.

In einer bevorzugten Ausführungsform gilt für die erfindungsgemäße Kombination, daß der COXII-Inhibitor (**Verbindung B**) ausgewählt ist aus:

Celecoxib, Rofecoxib, Etoricoxib, Valdecoxib, Parecoxib, Etodolac, Meloxicam oder Nimesulide, insbesondere

Celecoxib oder Rofecoxib.

Die erfindungsgemäßen Wirkstoffombinationen sind toxikologisch unbedenklich, so dass ein weiterer Gegenstand der Erfindung ein Arzneimittel enthaltend eine erfindungsgemäße Wirkstoffkombination; sowie gegebenenfalls geeignete Zusatz- und/oder Hilfsstoffe oder Wirkstoffe; ist.

Geeignete Zusatz- und/oder Hilfsstoffe im Sinne dieser Erfindung sind alle dem Fachmann aus dem Stand der Technik bekannten Stoffe zur Erreichung galenischer Formulierungen. Die Auswahl dieser Hilfsstoffe sowie die einzusetzenden Mengen derselben hängen davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parenterale,

topische und inhalative Applikation Lösungen, Suspensionen, leicht rekonstituierbare Trockenzubereitungen sowie Sprays. Eine weitere Möglichkeit sind Suppositorien für die Anwendung im Rektum. Die Anwen dung in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen. Beispiele für Hilfs- und Zusatzmitteln für die oralen Applikationsformen sind Sprengmittel, Gleitmittel, Binder, Füllmittel, Formtrennmittel, gegebenenfalls Lösungsmittel, Geschmacksstoffe, Zucker, insbesondere Trägermittel, Verdünnungsmittel, Farbstoffe, Antioxidantien etc. Für Suppositorien können u.a. Wachse bzw. Fettsäureester und für parenterale Applikationsmittel Trägerstoffe, Konservierungsmittel, Suspensionshilfsmittel etc. verwendet werden. Die an Patienten zu verabreichenden Wirkstoffmengen variieren in Abhängigkeit vom Gewicht des Patienten, von der Applikationsart und dem Schweregrad der Erkrankung. Aus oral, rektal oder perkutan anwendbaren Zubereitungsformen können die erfindungsgemäßen Verbindungen verzögert freigesetzt werden. Bei der erfindungsgemäßen Indikation sind entsprechende Retard-Formulierungen, insbesondere in Form eines "Once-daily"-Präparats, das nur einmal am Tag eingenommen werden muß, besonders bevorzugt.

Weiter bevorzugt sind Arzneimittel, die wenigstens 0,05 bis 90,0 % der Wirkstoffkombination enthalten, insbesondere niedrige wirksame Dosierungen, um Neben- oder analgetische Wirkungen zu vermeiden. Üblicherweise werden 0,1 bis 5000 mg/kg, insbesondere 1 bis 500 mg/kg, vorzugsweise 2 bis 250 mg/kg Körpergewicht der Kombination appliziert. Ebenso bevorzugt und üblich ist aber auch die Applikation von 0,01 - 5 mg/kg, vorzugsweise 0,03 bis 2 mg/kg, insbesondere 0,05 bis 1 mg/kg Körpergewicht.

Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzungzu bilden, die Wirkstoff in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzungwird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Da es sich als besonders bevorzugt herausgestellt hat, wenn der COX II-Inhibitor vor der Gabe des ausgewählten Analgetikums (Verbindung A) gegeben wurde, insbesondere mindestens 30 Minuten, vorzugsweise 1 Stunde vorher, sind galenische Formulierungen mit einem entsprechenden Freisetzungsprofil besonders bevorzugt. Ein Beispiel hierfür könnte z.B. ein osmotisch getriebenes Freisetzungssystem zum Erreichen einer verzögerten Freisetzung der Verbindung A mit einem den COX III-Inhibitor enthaltenden und entsprechend früher freisetzenden Überzug sein. Bei einem solchen, bevorzugt oralen, Freisetzungssystem ist mindestens eine, bevorzugt sämtliche, Oberfläche/n des Freisetzungsystems, bevorzugt die, die mit dem Freisetzungsmedium in Kontakt steht/stehen oder stehen könnte/können, semipermeabel, vorzugsweise mit einem semipermeablen Überzug ausgestattet, so dass die Oberfläche/n für das Freisetzungsmedium durchlässig, für den Wirkstoff, Verbindung A, aber im wesentlichen, bevorzugt vollständig, undurchlässig ist/sind, wobei die Oberfläche/n und/oder gegebenenfalls der Überzug wenigstens eine Öffnung zur Freisetzung des Wirkstoffes, Verbindung A, aufweist/aufweisen. Darüberhinaus ist/sind eben die Oberfläche/n, die mit dem Freisetzungsmedium in Kontakt steht/stehen, mit einem den COX II-Inhibitor enthaltenden und freisetzenden Überzug versehen. Vorzugsweise ist darunter ein System in Tablettenform mit einer Abgabeöffnung, einem osmotischen Arzneimittelkern, einer semipermeablen Membran und einem polymeren Teil, der Druck ausübt, zu verstehen. Ein gutes und bevorzugtes Beispiel für ein solches System ist dabei das OROS®-System der ALZA Corporation, USA, deren Internetauftritt oder andere Produktinformationen Einzelheiten über das OROS®-System enthält. Insbesondere sind dies auch

das OROS® Push-Pull™-System, das OROS® Delayed Push-Pull™-System, das OROS® Multi-Layer Push-Pull™-system, das OROS® Push-Stick System, bzw. auch in bestimmten Fragestellungen das L-OROS™. Ausführungsformen und Beispiele der konkreten Herstellung osmotisch getriebener Freisetzungssystem sind den US Patenten US 4,765,989, US 4,783,337 und US 4,612,008 zu entnehmen, die vollinhaltlich Bestandteil der Beschreibung dieser Erfindung sind.

Ein anderes Beispiel ist eine Gel-Matrix-Tablette, wie die von Penwest Pharamceuticals (beispielsweise unter TimeRX) entwickelten Produkte. Geeignete Beispiele finden sich in der US 5,330,761, US 5,399,362, US 5,472,711 und US 5,455,046. Insbesondere geeignet ist eine retardierende Matrixformulierung, mit einer inhomogenen Verteilung der Wirkstoffkombination, wobei der COX-II-Inhibitor vermehrt im äußeren Bereich (dem am schnellsten mit dem Freisetzungsmmedium in Kontakt kommenden Teil) der Matrix und das ausgewählte Analgetikum (Verbindung A) vermehrt im Inneren der Matrix zu finden ist. Bei Kontakt mit dem Freisetzungsmedium quillt die äußere Matrixschicht zuerst (und relativ schnell) auf und setzt zunächst den COX III-Inhibitor frei, gefolgt von der deutlich retardiert(er)en Freisetzung der Verbindung A. Beispiele für eine entsprechende Matrix sind Matrices mit 1 bis 80 Gew.-% eines oder mehrerer hydrophiler oder hydrophober Polymere als pharmazeutisch annehmbaren Matrixbildner. Ein weiteres Beispiel für eine geeignete Matrix ist der DE 33 09 516 A1 zu entnehmen.

Ein weiterer Gegenstand der Erfindung ist die Verwendung einer erfindungsgemäßen Wirkstoffkombination zur Behandlung von Schmerz, insbesondere neuropathischem, akuten, chronischem, viszeralem oder Krebsschmerz.

Weiter betrifft die Erfindung auch ein Verfahren zur Behandlung von Schmerz, bei dem die erfindungsgemäße Wirkstoffkombination verwendet wird.

Ein weiterer bevorzugter Gegenstand der Erfindung ist dabei eine Therapie, insbesondere ein Therapieschema, bei dem zur Behandlung von Schmerz der COX III-Inhibitor vor der Gabe des ausgewählten Analgetikums (Verbindung A) gegeben wird, da sich dies als besonders günstig herausgestellt hat. Besonders bevorzugt ist dabei eine Gabe des COX II-Inhibitors mindestens 30 Minuten, vorzugsweise 1 Stunde vor der Gabe der Verbindung A. Dies kann beispielsweise direkt durch den behandelnden Arzt erfolgen, aber auch (z.B. nach ärztlicher Anweisung) durch den Patienten selber. Dabei kann die Wirkstoffkombination auch getrennt in verschiedenen Arzneizubereitungen, insbesondere auch in verschiedenen Arzneipackungen vorliegen. Möglich und auch eine günstige Ausführungsform dieser Erfindung wäre ein Kit, bei dem die Wirkstoffe der erfindungsgemäßen Wirkstoffkombination zwar räumlich getrennt aber in einer Anbietungsform, beispielsweise einer Arzneimittelpackung, vorliegen. Entsprechend ist ein solcher "Kit" auch eine bevorzugte Ausführungsform der Erfindung.

**Beispiele:**

Die folgenden Beispiele sollen die Erfindung erläutern, ohne daß der Gegenstand der Erfindung darauf beschränkt wäre.

**Beispiel 1: Supra-additive Wirkung im Randall-Selitto-Test:**

Nachfolgend sind die auf ihre Wirksamkeit getesteten Verbindungen aufgelistet. Die Nummerierung wird in den Beispielen und den Abbildungen gebraucht:

**Verbindung A:**

| Name | Verbdg. Nr. |
|---|---|
| (1R,2R)-3-(2-Dimethylaminomethyl-cyclohexyl)-phenol;Hydrochlorid | **1** |
| (1R,2R)-3-(3-Dimethylamino-1-ethyl-2-methyl-propyl)- phenol; Hydrochlorid | **2** |
| (1S,2S)-3-(3-Dimethylamino-1-ethyl-2-methyl-propyl)-phenol; Hydrochlorid | **3** |
| (1RS,3RS,6RS)-6-Dimethylaminomethyl-1-(3-[methoxy-phenyl)-cyclohexane-1,3-diol; Hydrochlorid | **4** |

**COX II-Inhibitoren:**

| Name | Verbdg. Nr. |
|---|---|
| Celecoxib | **101** |
| Rofecoxib | **102** |
| Celecoxib in Form des Fertigarzneimittels CELEBREX® | **103** |
| Rofecoxib in Form des Fertigarzneimittels VIOXX® | **104** |

**Abbildung 1 und 2:** Supra-additive Wirkung der Kombination der ausgewählten analgetischen Verbindung Nr. 2 (Verbindung A) mit den COX-II-Inhibitoren im nachfolgend beschriebenen Randall-Selitto-Test bei zeitversetzter Applikation. Die Cox-Inhibitoren (Verbindungen 104 oder 103) wurden Ratten 1 h vor der Applikation Verbindung Nr. 2 p.o. gegeben. AUD = Area under Data. %MPE = % des maximal möglichen Effekts ("Maximal Possible Effect"), wobei der maximal möglicher Effekt = 100% ist.

**Abbildung 3:** Supra-additive Wirkung der Kombination der ausgewählten analgetischen Verbindung Nr. 4 (Verbindung A) mit dem COX-II-Inhibitor Verbindung Nr. 103, im Randall-Selitto-Test bei zeitversetzter Applikation im Zeitraum von 75 bis 90 min nach der Applikation der Verbindung 4. Der Cox-Inhibitor wurde Ratten 1 h vor der Applikation der Verbindung 4 p.o. gegeben..

**Abbildung 4:** Supra-additive Wirkung der Kombination der ausgewählten analgetischen Verbindung Nr. 1 (Verbindung A) mit dem COX-II-Inhibitor Verbindung Nr. 103, im Randall-Selitto-Test bei zeitversetzter Applikation. Weiterhin ist die Dauer der antinozizeptiven Wirkung bei der Kombination von Verbindung Nr. 103 und Verbindung Nr. 1 deutlich verlängert. Der Cox-Inhibitor wurde Ratten 1 h vor der Applikation der Verbindung Nr. 1 p.o. gegeben.

**Abbildung 5:** Supra-additive Wirkung der Kombination der ausgewählten analgetischen Verbindung Nr. 3 (Verbindung A) mit dem COX-II-Inhibitor Verbindung Nr. 103, im Randall-Selitto-Test bei zeitversetzter Applikation. Weiterhin ist die Dauer der antinozizeptiven Wirkung bei der Kombination von Verbindung Nr. 103 und Verbindung Nr. 3 deutlich verlängert. Der Cox-Inhibitor wurde Ratten 1 h vor der Applikation der Verbindung Nr. 3 p.o. gegeben

**Beispiel 2: Writhing-Test Maus**

Der Writhing-Test stellt eine Modifikation der Methode von Schmauss C and Yaksh TL, J Pharmacol Exp Ther 228: 1-12 (1984) und ein Modell für den Akutschmerz dar.

Die Tiere erhalten als Schmerzstimulus 9% Essigsäure intraperitoneal appliziert. Diese wird 30min (Testsubstanz p.o.) oder 10min (Testsubstanz i.v.) nach der Gabe der Testsubstanz appliziert. Registriert werden die Schmerz-induzierten Writhingreaktionen 10-35 min nach der Essigsäure-Gabe. Da die Ratte selten die klassische Writhingreaktion der Maus zeigt wohl aber andere sehr deutliche Schmerzäußerungen, erfolgt die Bewertung durch eine Score-Einteilung. Schmerzäußerungen wie z.B. Schiefneigung des Körpers beim stehenden Tier, häufiges Putzen des Bauches oder Katzenbuckel ohne Abstrecken der Hintergliedmaßen gehen so in die Bewertung ein.
Verglichen werden die substanzbehandelten Tiere mit einer Kontrollgruppe, die phys. Kochsalzlösung erhielt. Die Gruppengröße beträgt 10 Tiere (n=10).

Das Ergebnis der Wirkstoffkombination von Verbindung Nr. 2 mit den COX II -Inhibitoren 101, 102, 103 und 104 ist Tabelle 1 zu entnehmen.

**Beispiel 3: Formalin-Test**

Der Formalin Test (Dubuisson, D. and Dennis, S.G., 1977, Pain, 4, 161 - 174) stellt ein Modell für den akuten sowie den chronischen Schmerz dar. In den hier vorgestellten Untersuchungen wurde die chronische Schmerzkomponente ausgewertet.

Durch eine einmalige Formalin-Injektion in die dorsale Seite einer Hinterpfote wird bei freibeweglichen Versuchstieren eine biphasische nociceptive Reaktion induziert, die durch Beobachtung von drei deutlich voneinander unterscheidbaren Verhaltensmustern erfaßt wird.

Formalin wird mit dem Volumen von 20 μl und einer Konzentration von 1 % subkutan in die dorsale Seite der rechten Hinterpfote jedes Tieres appliziert. Die spezifischen Verhaltensänderungen, wie Anheben und Schütteln der Pfote, Gewichtsverlagerungen des Tieres sowie Beiss- und Leckreaktionen werden bis 60 min nach Formalinapplikation kontinuierlich beobachtet und registriert. Die Verhaltensänderungen werden unterschiedlich gewichtet (Score 0-3) und eine Pain-Rate (PR) errechnet mit folgender Formel:

$$PR = [(T_0 \times 0) + (T_1 \times 1) + (T_2 \times 2) + (T_3 \times 3) / 180.$$

Dabei entsprechen $T_0$, $T_1$, $T_2$, $T_3$ jeweils der Zeit in Sekunden, in der das Tier die Verhaltensweisen 0, 1, 2, oder 3 zeigte. Die Gruppengröße beträgt 10 Tiere (n=10).

Das Ergebnis der Wirkstoffkombination von Verbindung Nr. 2 mit den COX II -Inhibitoren 101, 102, 103 und 104 ist Tabelle 1 zu entnehmen.

**Beispiel 4: Randell-Selitto-Test**

Der Randall-Selitto-Test nach Randall und Selitto (Arch. Int. Pharmacodyn., 1957, 111:409-419) stellt ein Modell für den Entzündungsschmerz dar.

Durch Injektion von 0,1 ml 20 %ige Bäckerhefe-Suspension ventral in eine Hinterpfote wird ein Ödem induziert, an dem 4h später durch kontinuierlich ansteigendem Druck mit einem Stempel (2 mm Spitzendurchmesser) ein Schmerz erzeugt wird. Der zu bestimmende Messwert und gleichzeitig auch der Endpunkt des Schmerztestes ist der Druck bei dem die Vokalisationsreaktion des Tieres auftritt. Berechnet wird der prozentuale maximale mögliche Effekt ("maximal possible effect", %MPE). Dabei beträgt der maximale Druck des Stempels 250g. Die Gruppengröße beträgt n=10.

Die Ergebnisse der Versuche sind den Abbildungen 1 bis 4 sowie den nachfolgenden Tabellen 1 bis 4 zu entnehmen.

**Auswertung:**

Die Berechnung des theoretischen additiven Wertes erfolgt durch die Addition der Mittelwerte aus den Einzeluntersuchungen von Testsubstanz und COX-II Inhibitor. Die entsprechende Standardabweichung des theoretischen additiven Mittelwertes wurde aus der Addition der Varianzen berechnet.

Die ausgewählten Analgetika (Verbindung A) zeigten in allen oben aufgeführten Tiermodellen eine mittelstarke bis starke Hemmung der Nociception bzw. der Hyperalgesie.

Die simultane Applikation von einem selektiven COX-II-Inhibitor (Rofecoxib oder Celecoxib) mit der Verbindung 1 führte in den aufgelisteten Versuchen, also sowohl im Akut-, im chronischem- und im EntzündungsSchmerz (Tab. 1) zu einer Wirkverstärkung im Vergleich zur theoretischen errechneten rein additiven Wirkung. Hervorzuheben ist ein deutlicher wirkungssteigender supraadditiver Effekt bei zeitversetzter Gabe von COX-III-Inhibitor und den ausgewählten Analgetika (Verbindung A) Verbindungen Nr. 1, 4, 2 und 3 (Randall-Selitto-Test). Bei diesen Versuchen wurde entweder Rofecoxib oder Celecoxib eine Stunde vor der Applikation des ausgewählten Analgetikums (Verbindung A) gegeben. In einigen Untersuchungen wurde Rofecoxib als Fertigpräparat Vioxx und Celecoxib als Celebrex appliziert.

Die Ergebnisse ausgewählter Untersuchungen hier beispielsweise mit der Verbindung Nr. 1 sind in der nachfolgenden Tabelle (Tabelle 1) zusammengefasst. Fixed ratio combination: equieffektive Dosierung der beiden Substanzen, errechnet aus dem Verhältnis der jeweiligen ED50-Werte. Fixed dose combination: feste Dosierungen der jeweiligen Substanzen.

Tabelle 1:

| Test (Tiermodell) | Verbindgn. Nr. / Kombinat. (Vbdg. A + COX II) | Art der Applikation | Ratio der Verbindung A (1) zum COX-II-Inhibitor (2) 1: | 2: | Dosierung der Verbindung A (1) und des COX-II-Inhibitors (2) [mg/kg] 1: | 2: | % Wirkung / Ed50 [mg/kg] der Verbindung A | % Wirkung / Ed50 [mg/kg] des COX-II-Inhibitors | % Wirkung / Ed50 [mg/kg] der Kombination (theoretischer additiver Wert) | % Wirkung / Ed50 [mg/kg] der Kombination (experimentell. Wert) |
|---|---|---|---|---|---|---|---|---|---|---|
| **Fixed ratio combination** | | | | | | | | | | |
| Writhing (Ratte) | 2 + 102 | p.o. | 1 | 2 | - | - | $ED_{50}$: 33,0 | $ED_{50}$: 60,1 | $ED_{50}$: 47,0 | $ED_{50}$: 23,3 |
| Formalintes (Maus) | 2 + 101 | p.o. | 47,3 | 52,7 | - | - | $ED_{50}$: 34,6 | $ED_{50}$: 38,5 | $ED_{50}$: 36,5 | $ED_{50}$: 22,8 |
| Formalintes (Ratte) | 2 + 101 | p.o. | 10,5 | 89,5 | - | - | $ED_{50}$: 18,0 | $ED_{50}$: 154 | $ED_{50}$: 86,0 | $ED_{50}$: 60,6 |
| | | | | | | | | | | |
| **Fixed dose combination:** | | | | | | | | | | |
| Randall-Selitto (Ratte) | 2 + 104 | p.o. | - | - | 31 | 215 | 13,3 | 11,2 | 24,5 | 29,7 |
| Randall-Selitto (Ratte) | 2 + 101 | p.o. | - | - | 31 | 215 | 13,3 | 9,8 | 23,1 | 29,6 |
| **Fixed dose combination (zeitversetzte Gabe; COX-Inhibitor 1h vor Verbindung A):** | | | | | | | | | | |
| Randäll-Selitto (Ratte) | 2 + 104 | p.o. | - | - | 31 | 215 | 20,0 | 13,0 | 33,0 | 45,7 |
| Randall-Selitto (Ratte) | 2 + 104 | p.o. | - | - | 46 | 215 | 32,6 | 13,0 | 45,6 | 59,8 |
| Randall-Selitto (Ratte) | 2 + 103 | p.o. | - | - | 31 | 215 | 20,0 | 17,3 | 37,3 | 60,9 |

(fortgesetzt)

| Fixed dose combination (zeitversetzte Gabe; COX-Inhibitor 1h vor Verbindung A): | | | | | | | | | | |
|---|---|---|---|---|---|---|---|---|---|---|
| Randall-Selitto (Ratte) | 2 + 103 | p.o. | - | - | 46 | 215 | 32,6 | 17,3 | 49,9 | 86,1 |

Tabelle 2 zeigt die Versuche gemäß Beispiel 4 mit Verbindung Nr 4.

**Tabelle 2:**

| Test (Tiermodell) | Verbindgn. Nr. / Kombinat. (Vbdg. A + COX II) | Art der Applikation | Ratio der Verbindung A (1) zum COX-II-Inhibitor (2) | | Dosierung der Verbindung A (1) und des COX-II-Inhibitors (2) [mg/kg] | | % Wirkung / Ed50 [mg/kg] der Verbindung A | % Wirkung / Ed50 [mg/kg] des COX-II-Inhibitors | % Wirkung / Ed50 [mg/kg] der Kombination (theoretischer additiver Wert) | % Wirkung / Ed50 [mg/kg] der Kombination (experimentell. Wert) |
|---|---|---|---|---|---|---|---|---|---|---|
| | | | 1: | 2: | 1: | 2: | | | | |
| **Fixed dose combination (zeitversetzte Gabe; COX-Inhibitor 1h vor Verbindung A):** | | | | | | | | | | |
| Randall-Selitto (Ratte) | 4 + 103 | p.o. | - | - | 68,1 | 215 | 85,4 15 - 75 min* | 16,7 15-75 min | 102,1 15 - 75 min | 109,3 15 - 75 min |
| Randall-Selitto (Ratte) | 4 + 103 | p.o. | - | - | 68,1 | 215 | 45,2 75 - 90 min* | 8,0 75 - 90 min | 53,2 75 - 90 min | 68,5 75 - 90 min |
| * = Ausgewerteter Zeitraum nach Gabe von Verbindung Nr. 4 (s. Abb. 2) | | | | | | | | | | |

Tabelle 3 zeigt die Versuche gemäß Beispiel 4 mit Verbindung Nr 1.

**Tabelle 3:**

| Test (Tiermodell) | Verbindgn. Nr. / Kombinat. (Vbdg. A + COX II) | Art der Applikation | Ratio der Verbindung A (1) zum COX-II-Inhibitor (2) | | Dosierung der Verbindung A (1) und des COX-II-Inhibitors (2) [mg/kg] | | % Wirkung / Ed50 [mg/kg] der Verbindung A | % Wirkung / Ed50 [mg/kg] des COX-II-Inhibitors | % Wirkung / Ed50 [mg/kg] der Kombination (theoretischer additiver Wert) | % Wirkung / Ed50 [mg/kg] der Kombination (experimentell. Wert) |
|---|---|---|---|---|---|---|---|---|---|---|
| | | | 1: | 2: | 1: | 2: | | | | |
| **Fixed dose combination (zeitversetzte Gabe; COX-Inhibitor 1h vor Verbindung A):** | | | | | | | | | | |
| Randall-Selitto (Ratte) | 1 + 103 | p.o. | - | - | 46,4 | 215 | 26,2 | 8,9 | 35,1 | 84,1 |

Tabelle 4 zeigt die Versuche gemäß Beispiel 4 mit Verbindung Nr 3.

**Tabelle 4:**

| Test (Tiermodell) | Verbindgn. Nr. / Kombinat. (Vbdg. A + COX II) | Art der Applikation | Ratio der Verbindung A (1) zum COX-II-Inhibitor (2) | | Dosierung der Verbindung A (1) und des COX-II-Inhibitors (2) [mg/kg] | | % Wirkung / Ed50 [mg/kg] der Verbindung A | % Wirkung / Ed50 [mg/kg] des COX-II-Inhibitors | % Wirkung / Ed50 [mg/kg] der Kombination (theoretischer additiver Wert) | % Wirkung / Ed50 [mg/kg] der Kombination (experimentell. Wert) |
|---|---|---|---|---|---|---|---|---|---|---|
| | | | 1: | 2: | 1: | 2: | | | | |
| **Fixed dose combination (zeitversetzte Gabe; COX-Inhibitor 1h vor Verbindung A):** | | | | | | | | | | |
| Randall-Selitto (Ratte) | 3 + 103 | p.o. | - | - | 21,5 | 215 | 22,8 | 8,9 | 31,7 | 96,4 |

**Beispiel 6: Parenterale Applikationsform**

10 g (1 R,2R)-3-(2-Dimethylaminomethyl-cyclohexyl)-phenol;Hydrochlorid und 20 g Celecoxib werden in 1 l Wasser für Injektionszwecke bei Raumtemperatur gelöst und anschließend durch Zugabe von NaCl auf isotone Bedingungen eingestellt.

## Patentansprüche

1. Wirkstoffkombination aus wenigstens einer analgetischen **Verbindung A** und wenigstens einem **COX II-Inhibitor**, **dadurch gekennzeichnet, dass** der **COX II-Inhibitor** ausgewählt ist aus Celecoxib oder Rofecoxib, mit **Verbindung A** ausgewählt aus:

   (1R,2R)-3-(3-Dimethylamino-1-ethyl-2-methyl-propyl)-phenol; Hydrochlorid und
   (1S,2S)-3-(3-Dimethylamino-1-ethyl-2-methyl-propyl)-phenol; Hydrochlorid,
   in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

2. Arzneimittel enthaltend eine Wirkstoffkombination gemäß Anspruch 1 sowie gegebenenfalls geeignete Zusatz- und/oder Hilfsstoffe oder weitere Wirkstoffe.

3. Verwendung einer Wirkstoffkombination gemäß einem der Ansprüche 1 oder 2 zur Herstellung eines Medikamentes zur Behandlung von Schmerz, insbesondere neuropathischem, akuten, chronischem, viszeralem oder Krebsschmerz.

4. Verwendung gemäß Anspruch 3, wobei der Schmerz ausgewählt ist aus Entzündungsschmerz.

## Claims

1. Active ingredient combination from at least one analgesic **Compound A** and at least one **COX-II inhibitor, characterised in that** the **COX-II inhibitor** is selected from celecoxib or rofecoxib, with **Compound A** selected from:

   (1R,2R)-3-(3-dimethylamino-1-ethyl-2-methyl-propyl)-phenol; hydrochloride and
   (1S,2S)-3-(3-dimethylamino-1-ethyl-2-methyl-propyl)-phenol; hydrochloride,
   in the illustrated form or in the form of its acids or its bases or in the form of its salts, in particular the physiologically compatible salts, or in the form of its solvates, in particular the hydrates.

2. Medicament containing an active ingredient combination according to claim 1 and, if necessary, suitable additives and/or excipients or other active ingredients.

3. Use of an active ingredient combination according to any one of claims 1 or 2 for the manufacture of a medicament for the treatment of pain, in particular neuropathic, acute, chronic, visceral or cancer pain.

4. Use according to claim 3, wherein the selected pain is inflammatory pain.

## Revendications

1. Combinaison de substances actives se composant d'au moins un composé A analgésique et d'au moins un inhibiteur de COX II, **caractérisée en ce que** l'inhibiteur de COX II est choisi parmi le Célécoxib ou le Rofécoxib, le composé A étant choisi parmi :

   le chlorhydrate de (1R,2R)-3-(3-diméthylamino-1-éthyl-2-méthyl-propyl)-phénol et
   le chlorhydrate de (1S,2S)-3-(3-diméthylamino-1-éthyl-2-méthyl-propyl)-phénol,
   sous la forme représentée ou sous la forme de leurs acides ou de leurs bases ou sous la forme de leurs sels, en particulier de leurs sels physiologiquement acceptables, ou sous la forme de leurs produits de solvatation, en particulier de leurs hydrates.

**2.** Médicament contenant une combinaison de substances actives selon la revendication 1 et, le cas échéant, des additifs et/ou auxiliaires appropriés ou d'autres substances actives.

**3.** Utilisation d'une combinaison de substances actives selon l'une des revendications 1 ou 2 pour la préparation d'un médicament destiné au traitement de la douleur, en particulier de la douleur neuropathique, aiguë, chronique, viscérale ou cancéreuse.

**4.** Utilisation selon la revendication 3, la douleur étant choisie parmi les douleurs inflammatoires.

**Abbildung 1:**

**Verbindung Nr. 2 1h nach Verbindung Nr. 104**
**Verbindung Nr. 2 1h nach Verbindung Nr. 103**

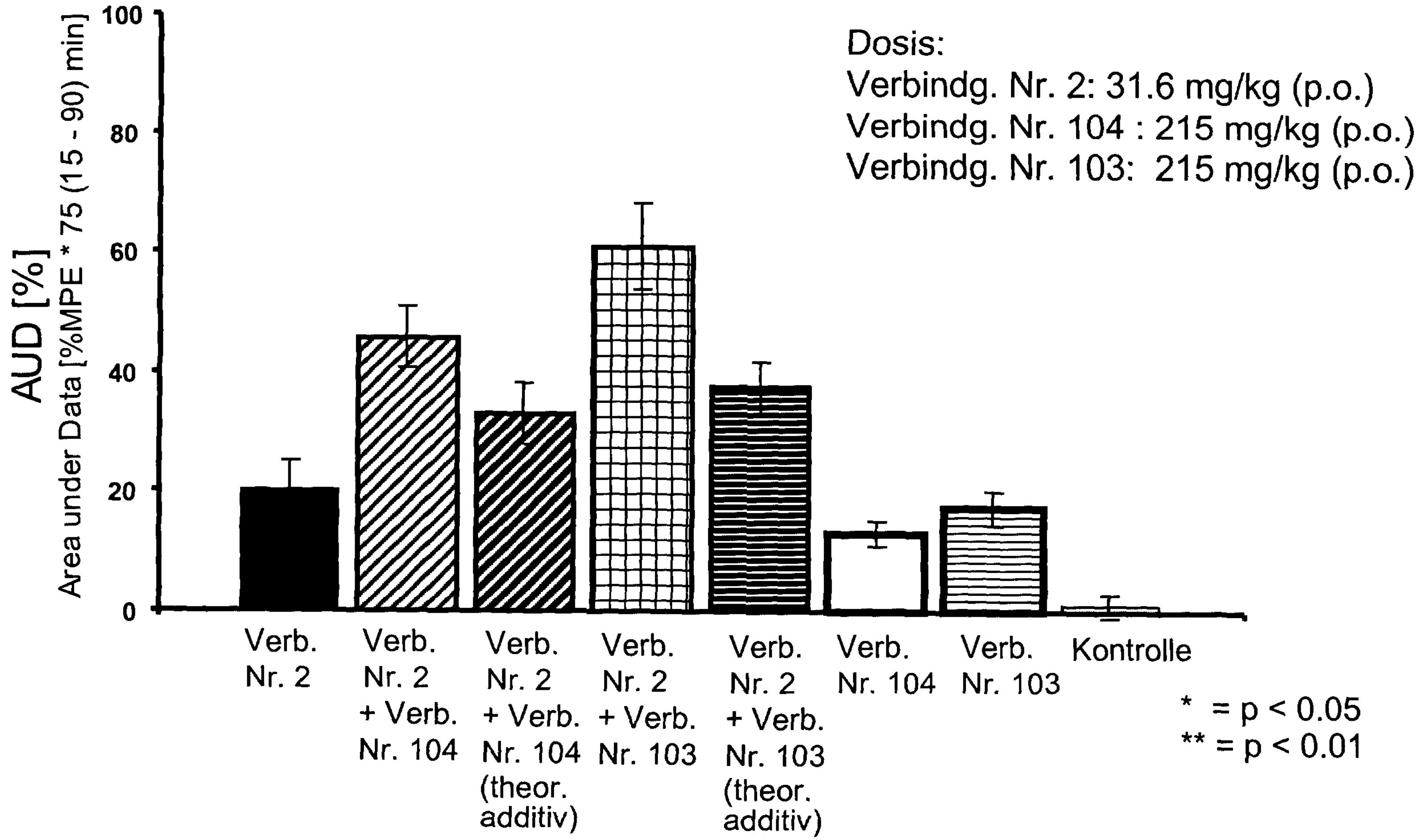

**Abbildung 2:**

**Verbindung Nr. 2 1h nach Verbindung Nr. 104**
**Verbindung Nr. 2 1h nach Verbindung Nr. 103**

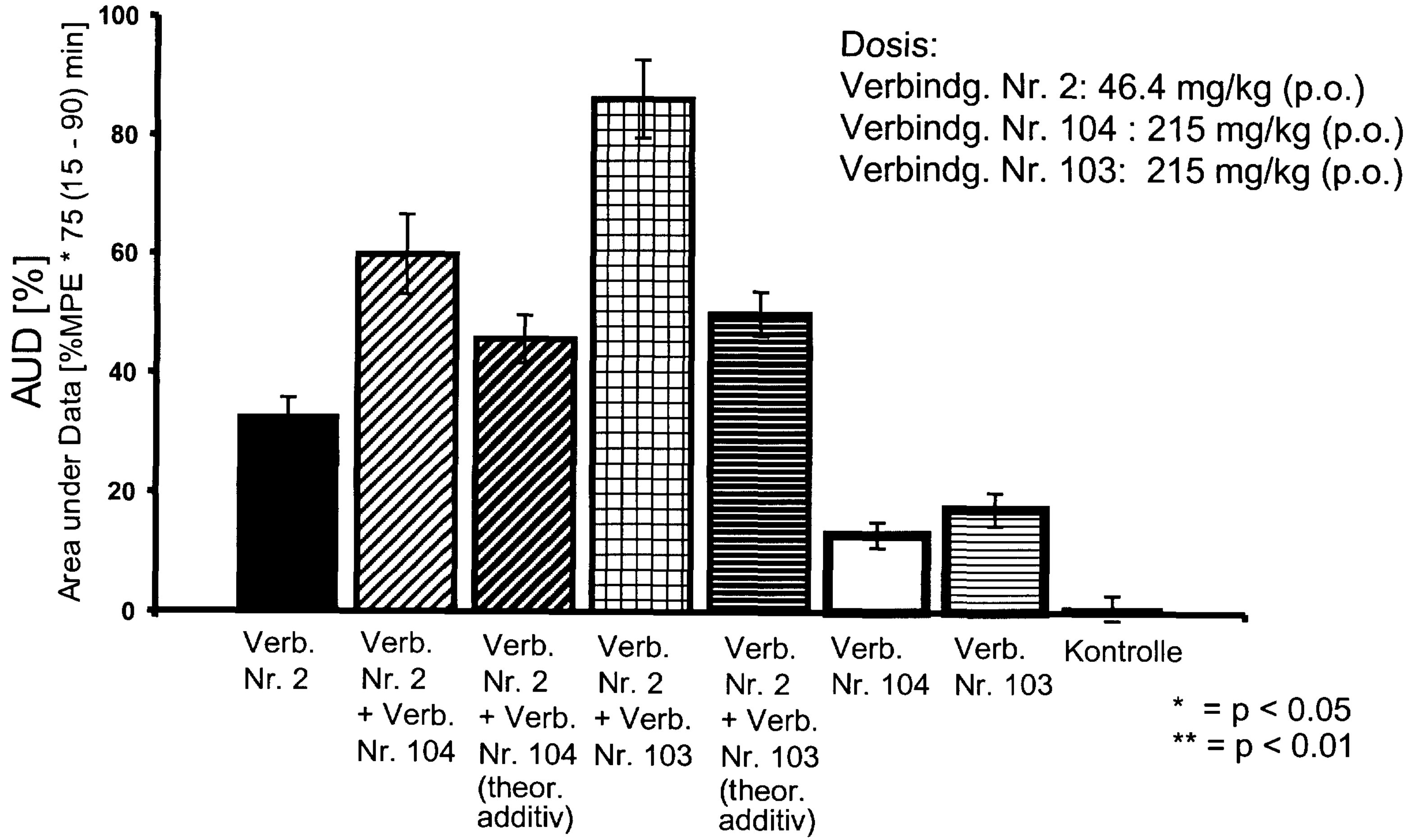

**Abbildung 3:**

**Verbindung Nr. 4 + Nr. 103**

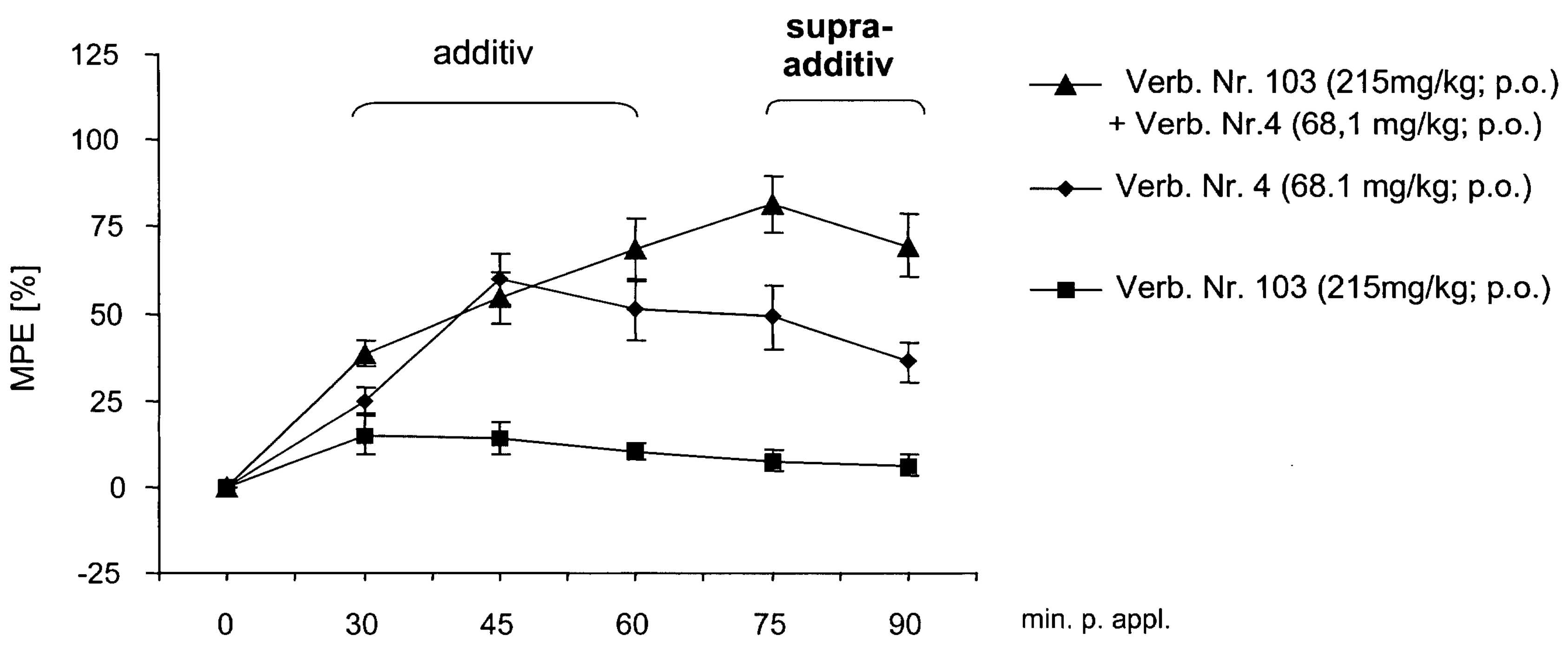


Anwendung von Verbindung Nr. 103 60 min vor Anwendung von Verbindung Nr. 4

**Abbildung 4:**

## Verbindung Nr. 1 + Nr. 103

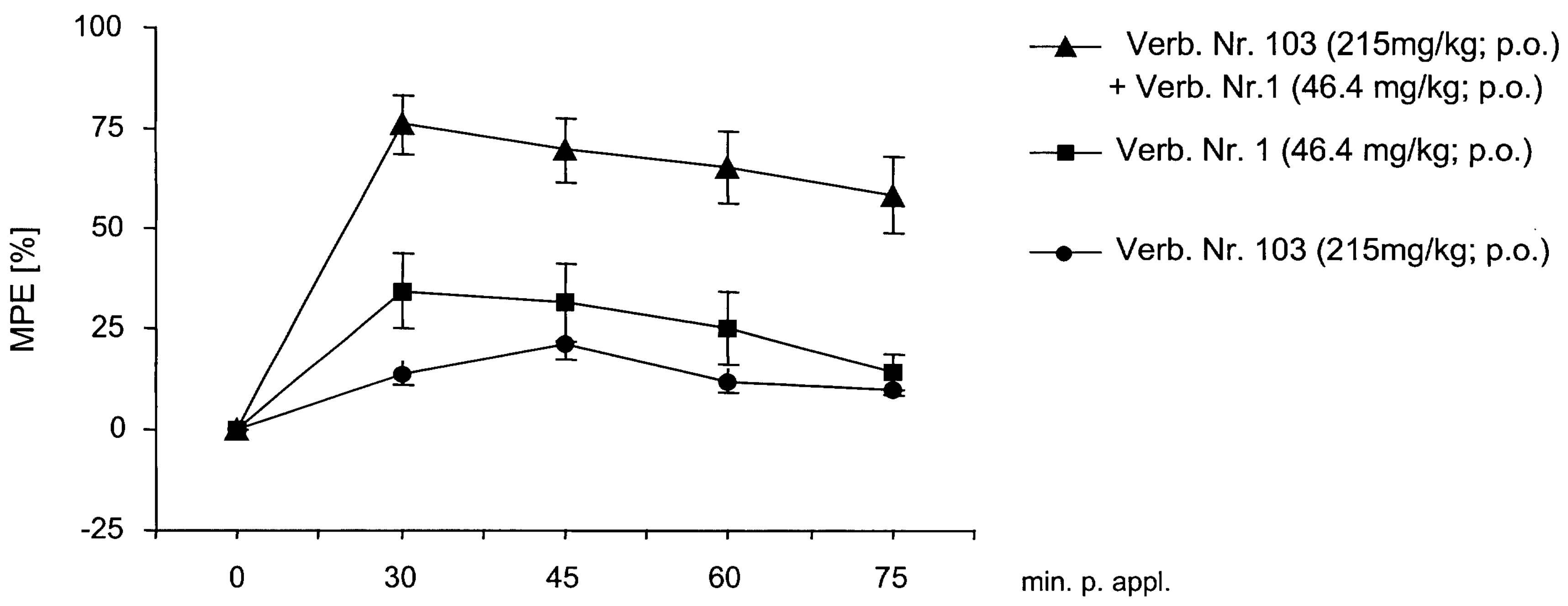


Anwendung von Verbindung Nr. 103 60 min vor Anwendung von Verbindung Nr. 1

**Abbildung 5:**

## Verbindung Nr. 3 + Nr. 103

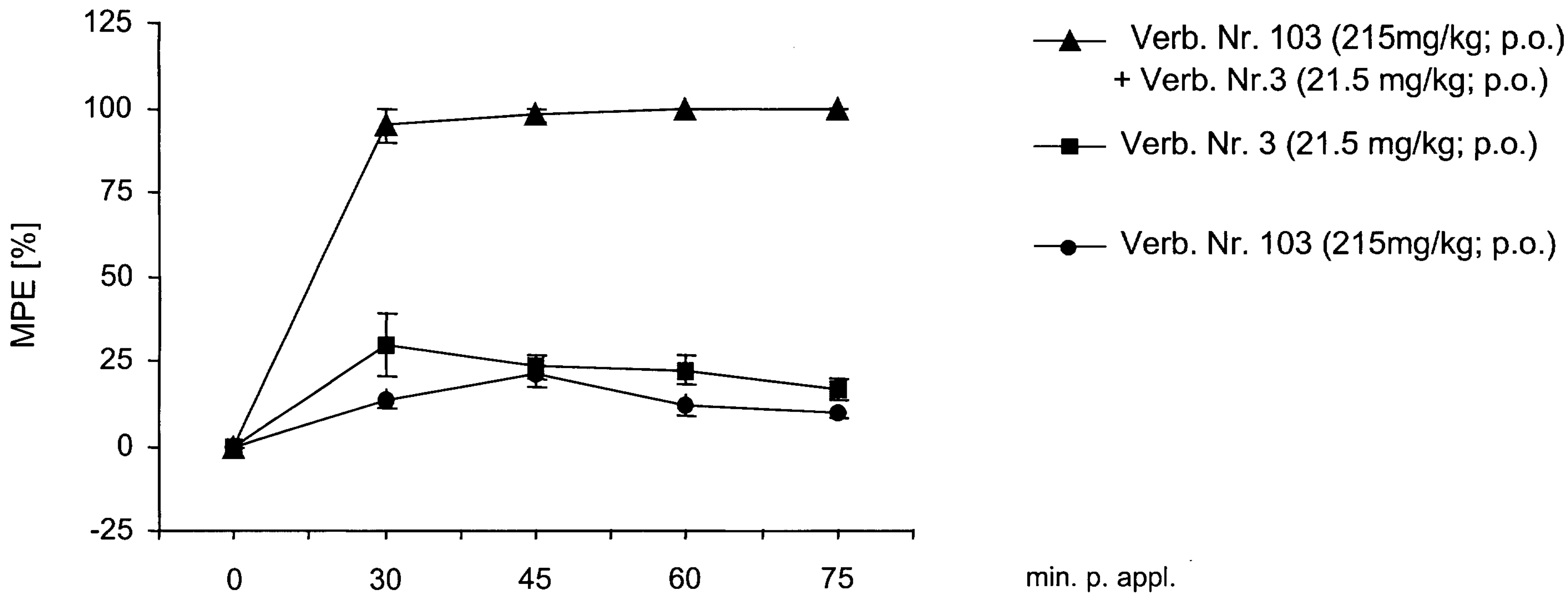


Anwendung von Verbindung Nr. 103 60 min vor Anwendung von Verbindung Nr. 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 4426245 A1
- DE 19525137 A1
- US 4765989 A
- US 4783337 A
- US 4612008 A
- US 5330761 A
- US 5399362 A
- US 5472711 A
- US 5455046 A
- DE 3309516 A1


### In der Beschreibung aufgeführte Nicht-Patentliteratur


- Remington's Pharmaceutical Sciences. Mack Publishing Company, 1985
- **VON SCHMAUSS C ; YAKSH TL.** *J Pharmacol Exp Ther,* 1984, vol. 228, 1-12
- **DUBUISSON, D. ; DENNIS, S.G.** *Pain,* 1977, vol. 4, 161-174
- **RANDALL ; SELITTO.** *Arch. Int. Pharmacodyn.,* 1957, vol. 111, 409-419